# EUROPEAN PATENT APPLICATION

(11) **EP 3 884 954 A1**
(43) Date of publication of application: **29.09.2021**
(21) Application number: 20900726.9
(22) Date of filing: 20.10.2020
(51) Int. Cl.: A61K 36/28, A61P 25/24, A23L 33/125

(54) **SUNFLOWER SEED COMPOSITION RICH IN PLEASANT HORMONE PRECURSOR AND ANTIDEPRESSANT USE THEREOF**

(30) Priority: 03.01.2020 CN 202010005574
(71) Applicant: Chacha Food Co., Ltd., Economic and Technological Development Zone Hefei, Anhui 230601 (CN)
(72) Inventor: SUN, Jin, Hefei, Anhui 230601 (CN); LU, Xiaomeng, Hefei, Anhui 230601 (CN); CHEN, Xianbao, Hefei, Anhui 230601 (CN); QI, Ce, Hefei, Anhui 230601 (CN)
(74) Representative: Gong, Jinping
(86) International application number: PCT/CN2020/122042
(87) International publication number: WO 2021/135520

(57) **Abstract**

The present invention discloses a sunflower seed composition rich in pleasant prohormone and anti-depression application thereof, and belongs to the field of extraction and application of natural products. Sunflower seeds used in the composition provided by the present invention are rich in tryptophan and have a high tryptophan/neutral amino acid ratio. After defatting treatment and chlorogenic acid removal treatment, and added part can increase a glycemic index. Glucose and vitamins for promoting absorption conversion of tryptophan and ω-3 fatty acids beneficial to nervous activities enable the sunflower seeds to have a better anti-depression effect. The sunflower seed composition of the present invention can improve concentrations of a monoamine neurotransmitter (serotonin) and dopamine in the brain and has a very good effect of ameliorating and treating depression. This composition uses natural sunflower seeds as raw material and is remarkable in anti-depression effect, rich in source and free of side effects.

## Description

### TECHNICAL FIELD

The present invention relates to a sunflower seed composition rich in pleasant prohormone and anti-depression application thereof, and belongs to the field of extraction and application of natural products.

### BACKGROUND

Depression is an emotional mental disease with general disorder of emotion, sleep, appetite, interest and thinking. Its morbidity, suicide rate, disability rate and mortality are very high, and it is not easy to cure because of repeated attacks. It not only causes great troubles and afflictions to patients, but also causes a huge economic loss to families and society. The study found that females are more likely to be depressed than males, and the incidence of depression is about 3 times that of males. The incidence of depression is on the rise due to the fast pace of life in modern society and the relatively high mental stress of people. The World Health Organization estimated that by 2020 depression would become the second most serious illness after heart disease. Many current synthetic antidepressants still have the shortcomings of slow onset of action, large side effect after long-term administration, and low reaction rate, etc. Today, the new trend in the treatment of depression resides in the use of natural products with better reaction rates and fewer side effects.

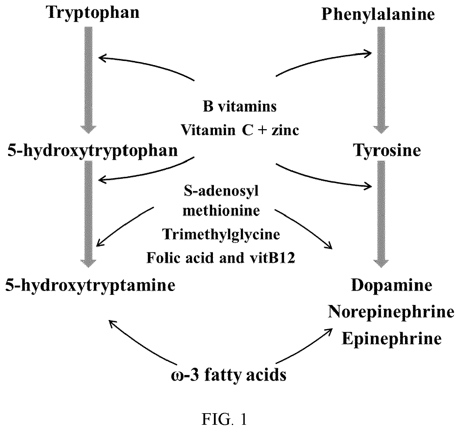

The brain 5-hydroxytryptamine (5-HT) participates in mood regulation (FIG. 1), and its precursor is tryptophan. Tryptophan (Trp) competes with large neutral amino acids (LNAAs: valine, leucine, isoleucine, phenylalanine, and tyrosine) to cross the blood-brain barrier and enters the brain by using an active protein shuttle. The ratio of Trp to LNAAs (TRP/LNAAs ratio) is considered to be the more sensitive availability index of brain tryptophan, which determines the extent to which tryptophan can enter the brain. A larger ratio means that Trp is more competitive and more easily enters the brain to play a role. One common cause of depression is imbalanced control of blood glucose levels in patients. Impaired control of blood glucose may cause dizziness, fatigue, irritability, depression and other symptoms, manifested by reduced sensitivity to insulin. Supplementing a proper amount of glucose can induce an increase in insulin levels in the blood. The Trp/LNAAs ratio increases with the increase in insulin levels, facilitating tryptophan absorption. In addition, vitamins play an auxiliary role in key enzymes controlling neurotransmitter production and balance. B vitamins such as B₆, B₁₂ and folic acid are components of co-enzymes in the human body, which are closely associated with amino acid metabolism. For example, methyl carboxylase synthesis required during the conversion of 5-hydroxytryptophan into 5-HT is highly dependent on B vitamins. When Trp is converted into serotonin, phenylalanine is converted into tyrosine and tyrosine is then converted into dopamine, norepinephrine, epinephrine, vitamins such as B6, B12 and folic acid are required to promote absorption and conversion of pleasant neurotransmitters. An increasing dietary intake of ω-3 fatty acids can increase the activity of central 5-HT, thereby ameliorating depression. When there are not enough ω-3 fatty acids in the brain, it is difficult to recognize 5-HT signals or activate brain cells, resulting in the loss of the 5-HT function in the brain and increasing the susceptibility to the cause of depression. ω-3 fatty acids can also affect neurotransmission by altering the fluidity of cell membrane and acting as precursors of inflammatory cytokines and eicosanoids, which may be associated with an anti-depressant effect. Persons supplemented with ω-3 fatty acids are less prone to depression, like building more serotonin in a factory, rather than merely increasing the utilization rate of existing serotonin, thereby improving the production efficiency and absorptivity of 5-HT.

Whey protein, sunflower seed protein and black sesame protein are rich in tryptophan and tyrosine, but the separation technology of whey protein in China started late, and there is no technology of comprehensive utilization of whey protein with independent intellectual property right available at present, while foreign technologies are protected by patents, so that resources such as whey protein in China mainly depend on imports. The planting history of black sesame in China is long, the nutritional value of sesame protein is high, the content of amino acids (except a low content of lysine) in the sesame protein is close to or reaches that of high-quality protein recommended by FAO/WHO compared with that of beef or casein, but the problems including the low yield of the black sesame protein, low processing scale and commodity conversion rate and protein denaturation easily occurring during the oil production process directly affect the utilization value and the functionality of the protein and cause the waste of the protein. Furthermore, allergens exist in the black sesame, and color and luster may be affected. In addition, the water-soluble nitrogen contained in sesame limits its utilization range. Sunflower seed protein is rich in tryptophan. Sunflower seed varieties with tryptophan content and tryptophan/neutral amino acid ratio meeting certain requirements can exert antidepressant effects similar to whey protein. Moreover, sunflower seeds are widely planted and abundant in China. It has higher application value as a food resource for regulating emotions.

### SUMMARY

The first object of the present invention is to provide an application of sunflower seeds or a sunflower seed composition in adjuvant treatment, alleviation or amelioration of depression, or preparation of a drug for treating depression. The sunflower seeds meet the requirement: the content of tryptophan is greater than or equal to 1.0 g/100 g sunflower seed protein.

In an implementation, the drug contains sunflower seed protein and a pharmaceutically acceptable carrier.

In an implementation, the composition contains 30-500 g of sunflower seeds, 25-150 mg of glucose, 200-1000 mg of ω-3 fatty acids and multi-vitamins.

In an implementation, the multi-vitamins include vitamin A, vitamin B₁, vitamin B₂, vitamin B₆, vitamin B₁₂ and folic acid.

In an implementation, the composition contains 30-500 g of sunflower seeds, 5-50 mg of vitamin B₆, 25-150 mg of glucose, 0.4-2 mg of folic acid, 5-50 µg of vitamin B₁₂ and 200-1000 mg of ω-3 fatty acids.

In an implementation, the composition contains 367 g of sunflower seed powder (providing 140 g of protein), 439.4 g of corn starch, 31.7 g of microcrystalline cellulose, 2.3 g of choline, 0.5 g of ω-3 fatty acids, 8 mg of vitamin A, 6 mg of vitamin B₁, 6 mg of vitamin B₂, 7 mg of vitamin B₆, 10 µg of vitamin B₁₂ and 2 mg of folic acid.

In an implementation, the content of tryptophan in the sunflower seeds is 1.0-3.0 g/100 g sunflower seed protein.

In an implementation, the sunflower seed protein is acquired after the sunflower seeds are subjected to defatting treatment and chlorogenic acid removal treatment in sequence.

In an implementation, the defatting treatment is deoiling by an oil press until the content of the oil is lower than 24%.

In an implementation, the chlorogenic acid removal process includes: placing the sunflower seeds into ethanol with a volume fraction of 50%-60% at a solid-liquid ratio of 1:10 to 1:14, ultrasonically extracting at 35-50°C for 25-35 min at an ultrasonic power of 140-160 W, performing vacuum filtration, and then vacuum-drying the product.

In an implementation, an intake of the sunflower seeds is greater than or equal to 1.5 g sunflower seeds/kg body weight.

In an implementation, an intake of the sunflower seed composition is greater than or equal to 3 g sunflower seed composition/kg body weight.

A second object of the present invention is to provide a composition. The composition contains 30-500 g of sunflower seeds, 5-50 mg of vitamin B₆, 25-150 mg of glucose, 0.4-2 mg of folic acid, 5-50 µg of vitamin B₁₂ and 200-1000 mg of ω-3 fatty acids. The sunflower seeds meet the requirement: the content of tryptophan is greater than or equal to 1.0 g/100 g protein.

The present invention further claims a method for the preparation of the sunflower seed composition. The method includes: subjecting the sunflower seeds to defatting treatment and chlorogenic acid removal treatment, and then mixing the sunflower seeds with 5-50 mg of vitamin B₆, 25-150 mg of glucose, 0.4-2 mg of folic acid, 5-50 µg of vitamin B₁₂ and 200-1000 mg of ω-3 fatty acids.

The present invention further claims the application of the sunflower seed protein in the preparation of food, drugs or health care products for preventing, treating, relieving or ameliorating depression. The sunflower seed protein meets the requirement: the content of tryptophan is greater than or equal to 1.0 g/100 g sunflower seed protein.

Beneficial effects: the sunflower seeds rich in tryptophan, phenylalanine and tyrosine are selected to be matched with glucose, tryptophan, vitamins such as vitamin B₆, vitamin B₁₂ and folic acid, and other ingredients, so that conversions of tryptophan into serotonin, phenylalanine into tyrosine and tyrosine into dopamine, norepinephrine and epinephrine, and absorption and conversion of pleasant neurotransmitters is promoted. The present invention proved by animal experiments that after mice were fed with sunflower seeds, the sucrose preference rate increased by 18.1-25.4%, and immobility time in tail suspension test and immobility time in forced swimming test were shortened by 28.2-40.5% and 32.6-35.3%, respectively. A measurement result of a hippocampal neurotransmitter content showed that by feeding the mice with the sunflower seeds, 5-HT and norepinephrine (NE) could increase by 22.61-36.68% and 10.64-70.48%, respectively, so that the anhedonia state of the mice can be relieved, the pleasant sensation is increased, and the depression and anxiety mood of the mice is effectively relieved.

### BRIEF DESCRIPTION OF DRAWINGS

FIG. 1 shows a relationship of 5-hydroxytryptamine participating in mood regulation;
FIG. 2 shows the effect of sunflower seeds on body weight of mice. The results are expressed as mean ± standard error of mean, and p is less than 0.05;
FIG. 3 shows the effect of sunflower seeds on a sucrose preference of mice. The results are expressed as mean ± standard error of mean, and p is less than 0.05;
FIG. 4 shows the effect of sunflower seeds on immobility time in tail suspension test (a) and forced swimming test (b). The results are expressed as mean ± standard error of mean, and p is less than 0.05;
FIG. 5 shows the effect of sunflower seeds on 5-HT (a), dopamine (DA, b), and norepinephrine (NE, c) in the hippocampus. The results are expressed as mean ± standard error of mean, and p is less than 0.05.

### DESCRIPTION OF EMBODIMENTS

A method for detecting the content of oil includes: analyzing the content of oil of sunflower seeds by using a full-automatic fat analyzer VELP, precisely weighing 5 g of sample into an extraction filter paper cylinder, adding 100 mL of petroleum ether into an extraction cup, and performing a full-automatic analysis process including immersion (60 min), removing (10 min), washing (60 min), recovery (30 min) and cooling (3 min) to measure the content of oil.

A method for detecting chlorogenic acid includes: accurately weighing 2.0 g of crushed sunflower seeds, adding an ethanol solution with a volume fraction of 60% at a solid-liquid ratio of 1:13 at 40°C, putting into an ultrasonic cleaner, regulating an ultrasonic power to be 150 W, performing ultrasonic extraction for 30 min, centrifuging to take 1 mL of supernate, and fixing a volume at 100 mL for measurement; accurately weighing 1.5 mg of chlorogenic acid standard product, fixing a volume with 50% ethanol in a 25 mL volumetric flask, and shaking up to prepare a standard solution; precisely sucking 0 mL, 1 mL, 2 mL, 3 mL, 4 mL and 5 mL of chlorogenic acid standard liquid, placing in 10-mL volumetric flasks, diluting to scales, measuring absorbance at 325 nm by using an ultraviolet spectrophotometer, drawing standard curves, and measuring the content of chlorogenic acid in the sunflower seeds.

The following further describes the present invention in conjunction with specific embodiments.

### Embodiment 1 Preparation of a sunflower seed protein

Sunflower seeds were deoiled by a cold-pressing method by using a Bestday ZYJ-9028 full-automatic oil press. The content of oil was reduced from 47.36% to 23.09% after deoiling. Then, chlorogenic acid was removed by using an ultrasonic-assisted alcohol extraction method. The ultrasonic-assisted alcohol extraction specifically includes: placing deoiled sunflower seeds into ethanol with a volume fraction of 60% at a solid-liquid ratio of 1:13, performing ultrasonic extraction at 40°C for 30 min at an ultrasonic power of 150 W, performing vacuum filtration, and then vacuum-drying filter residues to obtain sunflower seed powder. After detection, the content of chlorogenic acid reduced from 2.04% to 0.3% after ultrasonic-assisted alcohol extraction.

### Embodiment 2 Animal feeding and grouping

Six-week-old male C57BL/6J mice (SPF, purchased from Changzhou Cavens Experimental Animal Co., Ltd) were selected. The mice drunk and ate freely. After pre-feeding for 1 week, the mice were randomly divided into 6 groups (n=10 per group): normal control group (CON), model group (MOD), sunflower seed group (SFS), chlorogenic-acid-removed sunflower seed group (RSFS), whey protein group (WPC) and fish collagen protein group (FCP). The mice were fed for 4 weeks, all animals drunk freely, and dungs and urine were collected at the end of the 4th week. Ordinary daily rations of feeds were provided by animal feeding centres in the normal control group and the model group, and a feed formula of each group was as below.

CON group: every 1000 g contained 175 g (providing 140 g of protein) of soybean flour, 100.3 g of sucrose, 506.8 g of corn starch, 85 g of sunflower seed oil, 27.3 g of mineral additive (as shown in Table 1, prepared according to AIN-93M), 50 g of microcrystalline cellulose, 2.3 g of choline, 0.5 g of ω-3 fatty acids, and 10 g of multi-vitamins (including 8 mg of vitamin A, 6 mg of vitamin B₁, 6 mg of vitamin B₂, 7 mg of vitamin B₆, 10 µg of vitamin B₁₂ and 2 mg of folic acid).

The MOD group has the same feed formula as the CON group.

SFS group: based on the CON group, 175 g of soybean flour was replaced with 367 g of deoiled sunflower seed powder (providing 140 g of protein), the content of the corn starch was regulated to be 439.4 g, no sunflower seed oil was added, and the content of the cellulose was regulated to be 31.7 g, where the deoiled sunflower seed powder was treated as follows: sunflower seeds were deoiled by using a cold-pressing method through a Bestday ZYJ-9028 full-automatic oil press, the content of oil was reduced from 47.36% to 23.09% after deoiling, and then a residue was collected and vacuum-dried to obtain sunflower seed powder with a chlorogenic acid content of 2.04%.

The RSFS group has the same feed formula as the SFS group except that the deoiled sunflower seed powder was replaced with the sunflower seed powder prepared by using the method in Embodiment 1.

WPC group: based on the CON group, the soybean flour was replaced with 211 g of whey protein powder (providing 140 g of protein), and the content of the corn starch was regulated to be 474.1 g.

FCP: based on the CON group, the soybean flour was replaced with 164g of fish collagen protein powder (providing 140 g of protein), and the content of the corn starch was regulated to be 511 g.

**Table 1 Mineral mixture (AIN-93M-MX)**

| Ingredients | g/kg mix |
|---|---|
| Necessary mineral element | |
| Anhydrous calcium carbonate | 357 |
| Monopotassium phosphate | 250 |
| Sodium chloride | 74 |
| Potassium sulfate | 46.6 |
| Potassium citrate monohydrate | 28 |
| Magnesium oxide | 24 |
| Ferric citrate | 6.06 |
| Zinc carbonate | 1.65 |
| Manganese carbonate | 0.63 |
| Copper carbonate | 0.3 |
| Potassium iodate | 0.01 |
| Anhydrous sodium selenate/anhydrous sodium selenite | 0.01025 |
| Ammonium paramolybdate tetrahydrate/ammonium cis-molybdate tetrahydrate | 0.00795 |
| Sucrose Potential beneficial mineral element | 209.806 |
| Sodium metasilicate nonahydrate | 1.45 |
| Chromium potassium sulfate dodecahydrate | 0.275 |
| Boronic acid | 0.0815 |
| Sodium fluoride | 0.0635 |
| Nickel carbonate | 0.0318 |
| Lithium chloride | 0.0174 |
| Ammonium vanadate | 0.0066 |

Model construction: except the CON group, mice in other groups were fed in individual cages, induced to be depressive with reference to a chronic unpredictable mild stress (CUMS) model, and stressed alternatively twice every day for 21 days, and urine and dungs were collected one time in the end. The mice were subjected to a sucrose preference test and behavior rating after induction ended. Stress treatment was as below:
first day: the cages were inclined by 45 degrees for 2 h; tails were pinched for 3 min;
second day: the mice were placed in unpadded cages for 4 h; five mice were placed in the same cage for 3 h;
third day: the cages were inclined by 45 degrees for 2 h; no water or food for 12 h;
fourth day: rats were placed in padded cages for 3 h; water was added to empty cages by a depth of 0.8 cm to soak the rats for 4 h;
fifth day: tails were pinched for 3 min; five mice were placed in the same cage for 3 h;
sixth day: no water and food for 12 h; the rats were placed in padded cages for 3 h;
seventh day: water was added to empty cages to soak the rats for 4 h; the cages were inclined by 45 degrees for 2 h; and
operations in the 2nd week, the 3rd week and the 4th week were the same as above.

### Embodiment 3 Mouse behavior rating

### (1) Body weight recording

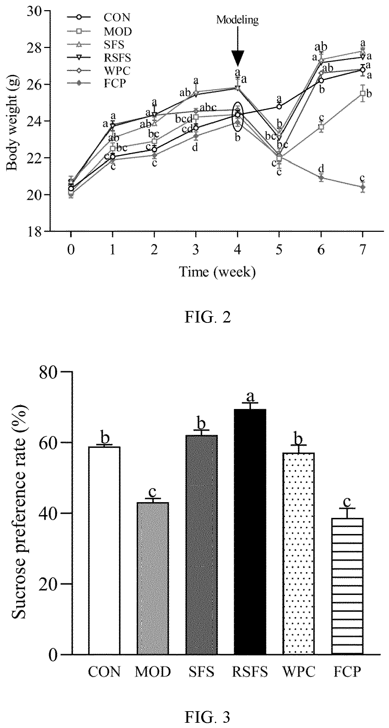

Mice were weighed once every week during an experimental process. As shown in FIG. 2, before modeling started (the 4th week), the body weights of mice in each group showed an increasing trend, and there was substantially no significant difference in body weight. In the 1st week of modeling, except for the CON group, the other groups showed a decreasing trend in body weight. A CUMS model was suddenly applied to the mice, which may cause eating discomfort and a decreasing trend in body weight. From the 5th week, the body weights of mice in each group recovered to increase except the FCP group where the body weights showed a decreasing trend. Compared with the MOD group, the body weights of mice in the SFS group and the RSFS group increased significantly (p is less than 0.05); the body weights of mice in the SFS group increased from 20.71 (± 0.94) g to 27.81 (± 0.57) g; the body weight of mice in the RSFS group increased from 20.65 (± 0.55) g to 27.48 (± 1.34) g; the body weight of mice in the MOD group increased at a lower rate from 20.16 (± 0.69) g to 25.52 (± 1.30) g, and the body weight of mice in the CON group showed an increasing trend from 20.34 (± 0.78) g to 26.78 (± 0.88) g during the whole period. It was found that the body weights of mice fed with deoiled sunflower seeds and deoiled and chlorogenic-acid-removed sunflower seeds increased more obviously. Therefore, sunflower seed protein has the activity of ameliorating depression behavior of mice and can effectively relieve depression.

### (2) Sucrose preference test

Sucrose preference test is a method for rating anhedonia, including a training period and a testing period. Training period: mice were given a bottle of 1% sucrose water and a bottle of pure water for 48 h, and the bottles were interchanged in this period to make mice adapt to drinking sucrose water. Testing period: after no water and food for 12 h, the mice underwent a sucrose preference test for 12 h, each bottle was weighed before and after the test, and a sucrose preference index (sucrose preference index=sucrose water consumption/total liquid consumption× 100%) was calculated. On the 2nd day after modeling ended, a sucrose preference test was carried out.

As shown in Table 2 and FIG. 3, compared with that of the CON group, the sucrose preference rate of mice in the MOD group showed an obvious decreasing trend (p is less than 0.05); compared with that of the MOD group, sucrose preference rates of mice in the SFS group and the RSFS group were increased (by about 19.0% and 26.4%, respectively), where the sucrose preference rate in the RSFS group was the most significant (p is less than 0.05), and increased more obviously (p is less than 0.05) compared with those of the positive control WPC group and the CON group. Therefore, it was shown that the sunflower seeds improved a sucrose preference rate of a mouse, increased the preference and pleasant sensation of the mouse to sucrose, and had a remarkable effect of ameliorating depression symptoms of the mouse.

**Table 2 The effect of sunflower seeds on sucrose preference of mice**

| Group | Sucrose preference rate/% |
|---|---|
| CON | 58.9±0.7^{b} |
| MOD | 43.2±2.9^{c} |
| SFS | 62.2±4.0^{b} |
| RSFS | 69.6±4.7^{a} |
| WPC | 57.1±5.6^{b} |
| FCP | 38.7±7.3^{c} |

The results are expressed as mean ± standard error of mean, and different superscripts represent significant differences (p is less than 0.05).

### (3) Open field test

Open field test (OFT) is a method for evaluating spontaneous activities of experimental animals in a new environment. The test is mainly used to evaluate whether sunflower seed protein has side effects on mice, e.g., affecting spontaneous activities of mice or causing anxiety-like behaviors to mice. In a dark room, an experimental box (40 × 40 × 30 cm) was placed. The experimental box was made of black transparent organic glass, a camera connected to a computer was disposed above the experimental box, and real-time images of mice in an experiment were traced and recorded to acquire behavior data. The test was carried out in 16 squares (10 × 10 cm). Four squares located in a central area were defined as a central region. To evaluate the spontaneous activities of mice, each mice was placed at a centre of a box and allowed to explore freely for 30 min.

As shown in Table 3, total distances of horizontal movement of mice in the MOD group and the FCP group had no significant difference from that of mice in the CON group and the WPC group, but compared with that of the MOD group, a total distance of movement of mice in the SFS group and the RSFS group was increased (p is less than 0.05); and the time and number of times the mice in the MOD group entered the central region were decreased compared with those of the CON group, and a distance of mice entering the central region did not differ from that of the CON group. Compared with those of the SFS group and the RSFS group, the time, distance and number of times the mice entered the central region were all remarkably decreased (p is less than 0.05). The results showed that the spontaneous activity of mice fed with sunflower seeds increased, compared with that of the MOD group, the anxiety state was effectively alleviated, the exploration to space was significantly enhanced, and the alleviation effect was more significant than that of the positive control whey protein. There was no significant difference in effect between chlorogenic-acid-removed sunflower seeds and sunflower seeds.

**Table 3 The effect of sunflower seeds on activities of mice in open field test**

| Group | Total distance/m | Central region time/s | Central region distance/m | Number of times the mice entered central region |
|---|---|---|---|---|
| CON | 72.87±14.81^{bc} | 294.38±17.87^{a} | 17.86±3.23^{ab} | 129.0±12.83^{b} |
| MOD | 66.64±3.06^{c} | 219.00±28.28^{b} | 13.86±1.57^{bc} | 93.0±6.08^{c} |
| SFS | 83.46±9.72^{ab} | 303.60±18.59^{a} | 17.86±2.28^{ab} | 132.0±10.92^{ab} |
| RSFS | 97.58±14.09^{a} | 303.57±6.99^{a} | 20.89±3.66^{a} | 148±6.40^{a} |
| WPC | 73.57±4.81^{bc} | 295.93±17.33^{a} | 15.59±1.05^{bc} | 118±13.30^{b} |
| FCP | 64.86±7.74^{c} | 198.62±17.28^{a} | 13.37±1.97^{c} | 95±12.53^{c} |

The results are expressed as mean ± standard error of mean, and different superscripts in the same columns represent significant differences (p is less than 0.05).

### (4) Tail suspension test

Tail suspension test (TST) refers to a test on a behavior that a mouse attempts to escape but cannot escape after tail suspension, thereby abandoning struggle and entering a depressive immobility state. The mice were inverted, the tail (1 cm from the tip of the tail) of the mice was pasted on a fixing device with adhesive tape, and the head of the mice was about 20 cm from a table top, so that the mice had no place to climb at the front, back, left and right, every two mice were separated by a baffle to hinder the sight and to prevent mutual interference, and the cumulative immobility time of each animal in the last 4 minutes within 6 min was observed (the immobility index indicated that mice had all limbs immobile except breathing).

### (5) Forced swimming test

Forced swimming test (FST) is a behavioral despair test. Animals are placed in a limited environment, and the animals struggle but cannot escape, thus providing an unavoidable stressing environment. After a while, the animals show a typical "immobile state". Mice were placed in a beaker with a height of 25 cm, a diameter of 18 cm, a water depth of 12 cm, and a water temperature of 25°C, placed in water for 6 min, and the cumulative immobility time in the last 4 min was recorded. Immobility means that mice stop struggling in the water, or are floating, with only small limb movements to keep the head floating on the water but no movement of the whole body.

**Table 4 The effect of sunflower seeds on immobility time in tail suspension test and forced swimming test**

| Group | TST time/s | FST time/s |
|---|---|---|
| CON | 123.1±11.5^{c} | 87.7±12.0^{c} |
| MOD | 163.0±9.2^{a} | 152.9±15.2^{a} |
| SFS | 117.0±15.8^{b} | 99.0±15.7b^{c} |
| RSFS | 97.0±13.4^{b} | 103.0±16.3b^{c} |
| WPC | 96.5±8.4^{c} | 114.6±19.3^{b} |
| FCP | 153.6±6.4^{a} | 168.8±8.2^{a} |

The results are expressed as mean ± standard error of mean, and different superscripts in the same columns represent significant differences (p is less than 0.05).

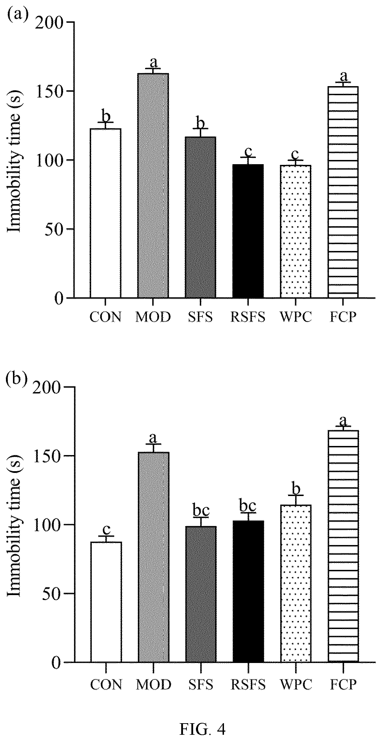

Tail suspension test and forced swimming test are classic tests to evaluate depression behaviors. As shown in FIG 4(a) and FIG 4(b), it can be seen that in TST and FST, compared with that of the CON group, the immobility time of mice in the MOD group was remarkably prolonged (p is less than 0.05) (32.4% and 74.3%). Compared with that of the MOD group, immobility time in the SFS group and the RSFS group was remarkably shortened (p is less than 0.05), the immobility time in the SFS group was shortened by 28.2% and 35.3% respectively, and immobility time in the RSFS group was shortened by 40.5% and 32.6% respectively. Results showed that mice in the MOD group exhibited an obvious behavior despair state in a limited environment, the "immobility time" increased significantly, while the immobility time of mice fed with sunflower seeds and chlorogenic-acid-removed sunflower seeds decreased significantly, showing an anti-depressant effect similar to whey protein, and indicating that sunflower seeds can effectively relieve anxiety.

### (6) Elevated plus maze test

The Elevated plus maze (EPM) test was a classic behavioral test to evaluate the anxiety of mice. A test apparatus consisted of two open arms and two closed arms with dimensions of 50 × 5 cm (length × width) in a crossed manner, where the closed arms were closed by opaque walls 30 cm in height, a central area was 5 × 5 cm in size and was not closed by walls, and the whole apparatus was 45 cm in height from the ground. The principle is that mice inherently like darkness and tend to stay in the closed arms, but the curiosity to a new object can drive the mice to enter the open arms, the conflict behaviors of inquiry and avoidance occur between the two choices, and hence the mice feel anxious. In addition, a suspended overhead may cause fear and restlessness in mice. Thus, the anxiety state of the mice can be evaluated by comparing the number of times the mice enter the open arms and the residence time.

It can be seen from Table 5 that the total numbers of entries of the CON group, the SFS group, the RSFS group and the WPC group was not significantly different from that of the MOD group, but the total number of entries of the SFS group and the RSFS group was more than that of the MOD group, and there were significant differences in the number of entries in the open arm, the residence time and the percentage of entry time compared with those of the MOD group, showing better performance. Therefore, the sunflower seeds can effectively ameliorate the depression symptoms of mice, and the chlorogenic-acid-removed sunflower seeds are more obvious in effect.

**Table 5 The effect of sunflower seeds on activities of mice in an elevated plus maze test**

| Group | Total number of times | Open arm | | |
|---|---|---|---|---|
| | | Number of entries | Residence time | Percentage of entry time/% |
| CON | 23.14±1.95^{b} | 9.00±2.19^{b} | 81.33±6.88^{a} | 37.75±8.63^{a} |
| MOD | 26.90±5.26^{ab} | 6.33±1.75^{c} | 47.76±4.40^{b} | 26.35±3.09^{c} |
| SFS | 28.75±5.57^{ab} | 12.83±0.75^{b} | 78.58±8.91^{a} | 35.92±3.53^{ab} |
| RSFS | 30.57±5.97^{a} | 9.86±0.90^{a} | 78.60±12.18^{a} | 36.85±5.15^{ab} |
| WPC | 27.44±6.23^{ab} | 9.00±1.91^{b} | 74.16±7.35^{a} | 30.32±5.17^{bc} |
| FCP | 25.70±4.92^{ab} | 6.67±1.63^{c} | 47.08±3.35^{b} | 25.55±5.03^{c} |

The results are expressed as mean ± standard error of mean, and different superscripts in the same columns represent significant differences (p is less than 0.05).

### Embodiment 4 Measurement of biochemical indexes

### (1) Measurement of hippocampal neurotransmitter

Contents of serotonin (5-HT), dopamine (DA) and norepinephrine (NE) in hippocampus were implemented strictly under the ELISA kit user guide.

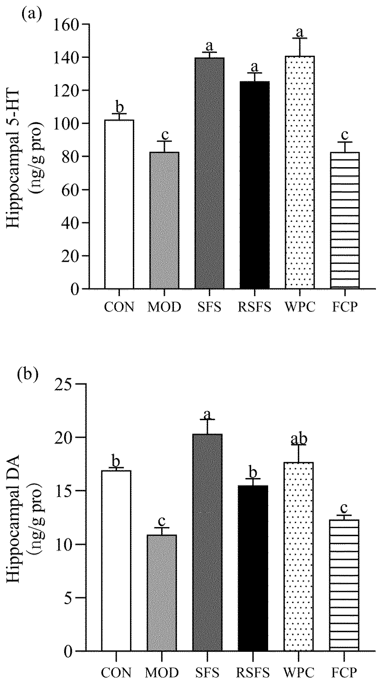

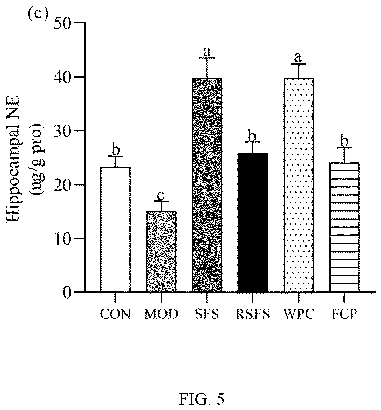

As shown in FIG. 5, compared with that of the CON group, the levels of 5-HT, DA and NE of the MOD group were remarkably reduced (p is less than 0.05) (by about 19.03%, 35.46% and 35.18% respectively). Compared with those of the CON group, the levels of 5-HT, DA and NE in the SFS group increased by 36.68%, 20.21% and 70.48% respectively; and contents of 5-HT and NE in the RSFS group increased by 22.61% and 10.64% respectively, and the level of DA changed a little, showing better performance than that of the MOD group. Although there was no significant difference between the two groups, the levels in the SFS group were slightly higher than those in the RSFS group. It was shown that sunflower seeds can increase the concentration of pleasant neurotransmitters in the brain, relieve anhedonia in mice, increase pleasant sensation, and effectively relieve depression and anxiety in mice.

It can be seen from the evaluation results of Embodiments 3-4 that the selected sunflower seeds can increase the body weight of mice and have a relatively better effect than the positive control whey protein, obviously improve the sucrose preference rate of the mice and the sucrose preference and the pleasant sensation of the mice, have the activity of ameliorating the depression behavior of the mice, and can effectively relieve the depressed mood.

The open field test showed that the sunflower seeds can enhance the spontaneous activity of the mice. Compared with that of the MOD group, the anxiety state was relieved effectively, the exploration to space was enhanced significantly, and the relief effect was more significant than that of the positive control whey protein; the sunflower seeds can shorten the immobility time of the mice in tail suspension test and forced swimming test, showing the anti-depression effect similar to that of whey protein; the results of the elevated plus maze test showed that the sunflower seeds can effectively ameliorate the anxiety behavior of the mice and relieve the depression of the mice under relatively high environmental stress.

The sunflower seeds can increase the concentration of pleasant neurotransmitters in the brain, relieve the anhedonia state of mice and increase the pleasant sensation. Therefore, the sunflower seeds can effectively relieve the depression behavior, can have an anti-depressant effect similar to that of whey protein, have the activity of relieving the depression and can effectively treat the depression, and the sunflower seeds have a better effect than the chlorogenic-acid-removed sunflower seeds.

Although the present invention has been described in conjunction with the preferred embodiments above, these embodiments are not intended to limit the present invention. Various changes and modifications can be made by those skilled in the art without departing from the spirit and scope of the present invention. Hence, the protection scope of the present invention should be defined by claims.

## Claims

1. Application of sunflower seeds or a sunflower seed composition in adjuvant treatment, alleviation or amelioration of depression, or preparation of a drug for treating depression, wherein the content of tryptophan in the sunflower seeds is greater than or equal to 1.0 g/100 g sunflower seed protein.

2. The application according to claim 1, wherein the composition contains 30-500 g of sunflower seeds, 25-150 mg of glucose, 200-1000 mg of ω-3 fatty acids and multi-vitamins.

3. The application according to claim 1 or 2, wherein an intake of the sunflower seeds is not less than 1.5 g sunflower seeds/kg body weight, or an intake of the sunflower seed composition is not less than 3 g sunflower seed composition/kg body weight.

4. A sunflower seed protein, wherein sunflower seeds are subjected to defatting treatment and chlorogenic acid removal treatment in sequence; the defatting treatment is deoiling by an oil press until the content of the oil is less than or equal to 24%; and the chlorogenic acid removal process comprises: placing the sunflower seeds into ethanol with a volume fraction of 50%-60% at a solid-liquid ratio of 1:(10-14), ultrasonically extracting at 35-50°C for 25-35 min at an ultrasonic power of 140-160 W, and then performing filtration and drying.

5. The sunflower seed protein according to claim 4, wherein the drying is vacuum drying.

6. The sunflower seed protein according to claim 4, wherein the content of tryptophan is greater than or equal to 1.0 g/100 g sunflower seed protein.

7. A composition containing the sunflower seed protein according to any one of claims 4-6.

8. The composition according to claim 7, wherein composition contains 25-150 mg of glucose, 0.4-2 mg of folic acid, 200-1000 mg of ω-3 fatty acids and multi-vitamins.

9. The composition according to claim 8, wherein the multi-vitamins comprise vitamin A, vitamin B₁, vitamin B₂, vitamin B₆, vitamin B₁₂ and folic acid.

10. Application of the sunflower seed protein according to any one of claims 4-6 or the composition according to any one of claims 7-9 in preparation of food, health care products or food additives for preventing, treating, relieving or ameliorating depression, wherein the sunflower seeds meet the requirement: the content of tryptophan is greater than or equal to 1.0 g/100 g sunflower seed protein.
